(19) Europäisches Patentamt
European Patent Office
Office européen des brevets

(11) Publication number: **0 115 091**
**A1**

# (12) EUROPEAN PATENT APPLICATION

(21) Application number: 83200158.0

(22) Date of filing: 27.01.83

(51) Int. Cl.[3]: C 07 C 103/52
A 61 K 37/02, C 07 D 207/16
C 07 D 277/06

(43) Date of publication of application:
08.08.84 Bulletin 84/32

(84) Designated Contracting States:
AT BE CH DE FR GB LI LU NL SE

(71) Applicant: ENI-Ente Nazionale Idrocarburi
P.le E.Mattei 1
I-00144 Roma(IT)

(72) Inventor: Zappelli, Piergiorgio
Via G. di Vittorio 3
I-00015 Monterotondo (Rome)(IT)

(72) Inventor: Eletti Bianchi, Giancarlo
Via Conca d'Oro 341
I-00141 Rome(IT)

(72) Inventor: Pappa, Rosario
Via S. Matteo 86/B
I-00015 Monterotondo (Rome)(IT)

(72) Inventor: Bassignani, Luciano

I-02036 Passo Corese(Rieti)(IT)

(72) Inventor: Rossodivita, Antonio
Via Mameli 13
I-00015 Monterotondo (Rome)(IT)

(72) Inventor: Re, Luciano
Via E. Mambretti 15
I-00194 Rome(IT)

(74) Representative: Roggero, Sergio et al,
Ing. Barzanò & Zanardo Milano S.p.A. Via Borgonuovo 10
I-20121 Milano(IT)

## (54) N-carboxyacyl derivatives of cyclic amino acids, useful as antihypertensives.

(57) This invention relates to compounds useful as active principles of formula I in the treatment of hypertension, and to processes for their preparation.

I :

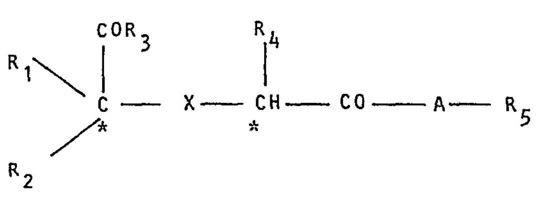

where

$R^1$ and $R^4$, equal or different, are hydrogen, alkyl substituted alkyl, aryl, aralkyl or alkyl substituted with an heterocyclic radical,

$R^2$ is hydrogen, alkyl, aryl or aralkyl;

$R^3$ and $R^5$, equal or different, are hydroxy, substituted hydroxy amino, substituted amino,

X is

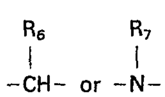

where $R^6$ and $R^7$, equal or different, are hydrogen, alkyl or aralkyl;

A is the residue

a)

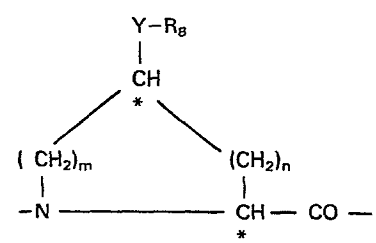

where n and m are 1 or 2 and m+n is 2 or 3, Y is either −O− or −S− and $R^8$ is aryl, a heterocyclic radical, acyl, arylsulphonyl, alkylsulphonyl, trialkylsilyl, nitro, cyclic saturated alkyl, cyclic or non-cyclic unsaturated alkyl, substituted alkyl, aralkyl; or alkyl substituted with a heterocyclic radical; when Y is −S−, $R^8$ can also be hydrogen or non-cyclic saturated alkyl;

./...

EP 0 115 091 A1

b)

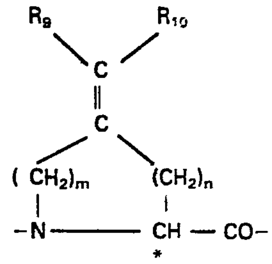

where m and n are 1 or 2 and m+n is 2 or 3;
$R^9$ and $R^{10}$, equal or different, are hydrogen, alkyl, substituted alkyl, aryl, aralkyl or alkyl substituted with an heterocyclic radical, and in which the alkyl portion can also comprise an amino or acylamino substituent;

c)

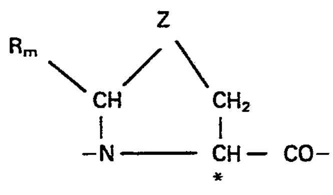

where Z is −S−, 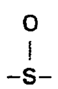 or 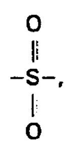,

and $R^{11}$ is hydroxy, substituted hydroxy, carboxy or substituted carboxy, eneoxycabonyl, aryloxycarbonyl, amino, substituted amino, substituted alkyl; the asterisks indicating asymmetric carbon atoms; and the pharmaceutically acceptable salts of said derivatives.

This invention relates to new compounds capable of reducing or removing angiotensin-dependent hypertension caused by the conversion of angiotensin I into angiotensin II by the angiotensin conversion enzyme in mammals.

The renin-angiotensin-aldosterone system has been recently recognised as one of the factors of primary importance in causing hypertension. The action of the renin enzyme on a blood plasma pseudoglobulin produces a decapeptide, namely angiotensin I, which is converted into the octapeptide angiotensin II by the angiotensin conversion enzyme. Angiotensin II exerts a powerful vasoconstricting action which increases the blood pressure.

The angiotensin conversion enzyme is also responsible for the inactivation of bradykinin, a nonapeptide of powerful hypotensive action. Thus an inhibiter of said enzyme can exert an antihypertensive action, both by inhibiting the formation of angiotensin II, and by inhibiting the destruction of bradykinin.

Compounds which inhibit the angiotensin conversion enzyme can be used for treating angiotensin-dependent hypertension such as renal hypertension and malignant hypertension. Compounds and methods have already been proposed for treating angiotensin-dependent hypertension. Reference should be made for example to USA patents Nos. 4,046,889 and 4,052,511, German patent application No. 2,752,719, Dutch patent application No. 78 098806, and European patent application No. 0,012,401, which relate to the use of amino acid derivatives in inhibiting the angiotensin conversion enzyme. The inhibiting power of the compounds described in the cited patents

and of the other products reported in the known art is often very high.

However, the only product at an advanced and documented stage in clinical trials on human beings for the purpose of its final use in therapy has in some cases shown toxic secondary effects (see for example Brit. Med. J. 2, 1680 (1979); Biol. Med. J. 3, 612 (1979); Lancet 2, 557 (1979); Lancet 1, 712 and 926 (1980)). The said product must be administered at high dosage in order to obtain an effective reduction in hypertension (N. Engl. J. Med. 298, 991 (1978)).

We have now discovered a particular class of N-glutaryl and N-[(carboxymethylamino)acetyl] derivatives of cyclic amino acids which are capable of reducing or removing hypertension caused by the conversion of angiotensin I into angiotensin II by the angiotensin conversion enzyme in mammals.

This class of derivatives constitutes the subject matter of the present invention. The derivatives according to the present invention can be chosen from those corresponding to the general formula

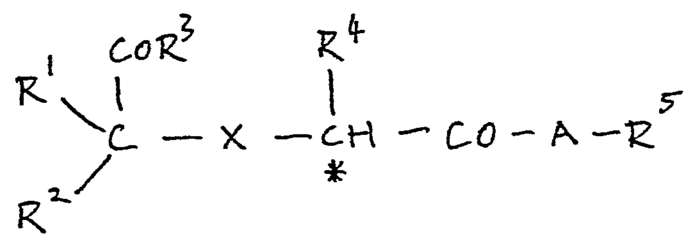

(I)

and from their pharmaceutically acceptable salts.

In said formula I, the symbols have the following meanings.

$R^1$ and $R^4$, which can be equal or different, are hydrogen, alkyl, haloalkylene, hydroxyalkylene, alkyloxyalkylene, aryloxyalkylene,

aminoalkylene, mono or di-alkylaminoalkylene, acylaminoalkylene, arylaminoalkylene, guanidinoalkylene, mercaptoalkylene, alkylthioalkylene, arylthioalkylene, carboxyalkylene, alkyloxycarbonylalkylene, carbomoylalkylene,aryl or alkyl substituted with an aryl or heterocyclic radical, and in which the alkyl portion can also comprise an amino or acylamino substituent;

$R^2$ is hydrogen, alkyl, aryl or arylalkylene;

$R^3$ and $R^5$, which can be equal or different, are hydroxy, alkyloxy, aryloxy, arylalkyleneoxy, amino, mono or di-alkylamino, arylalkyleneamino, hydroxyamino, dialkylaminoalkyleneoxy, acylaminoalkyleneoxy or acyloxyalkyleneoxy;

X is $-\overset{R^6}{\overset{|}{C}}H-$ or $-\overset{R^7}{\overset{|}{N}}-$ where $R^6$ and $R^7$, which can be equal or different, are hydrogen, alkyl or arylalkylene;

A is the residue

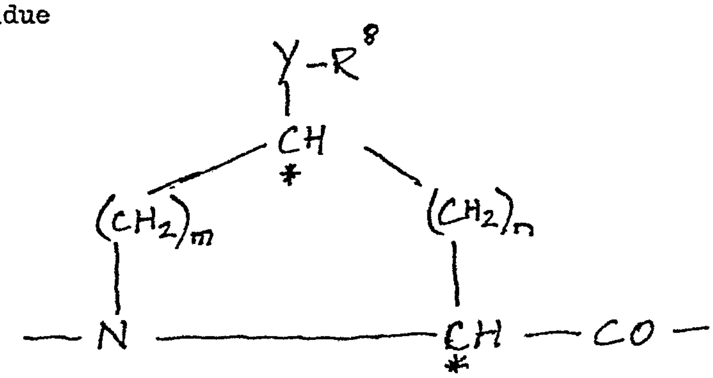

where n and m are 1 or 2 and m+n is 2 or 3, Y is either-O- or-S- and $R^8$ is aryl, a heterocyclic radical, acyl, arylsulphonyl, alkylsulphonyl, trialkylsilyl, nitro, cyclic saturated alkyl, cyclic or non-cyclic unsaturated alkyl, haloalkylene, hydroxyalkylene, alkyloxyalkylene, mercaptoalkylene, alkylthioalkylene, aminoalkylene, mono or di-alkylaminoalkylene, guanidinoalkylene, arylalkylene or alkyl substituted with a heterocyclic radical;

when Y is -S-, $R^8$ can also be hydrogen or non-cyclic saturated alkyl;

A can also be the residue

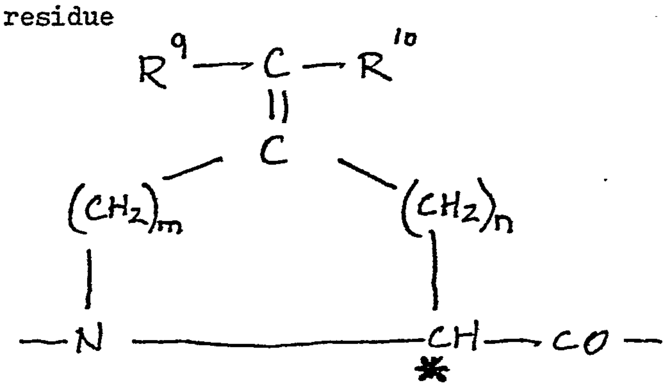

where m and n are 1 or 2 and m+n is 2 or 3;

$R^9$ and $R^{10}$, which can be equal or different, are hydrogen, alkyl, haloalkylene, hydroxyalkylene, alkyloxyalkylene, aryloxyalkylene, aminoalkylene, mono or di-alkylaminoalkylene, acylaminoalkylene, arylaminoalkylene, guanidinoalkylene, mercaptoalkylene, alkylthioalkylene, arylthioalkylene, carboxyalkylene, alkyloxycarbonyl-alkylene, carbamoylalkylene, aryl or alkyl substituted with an aryl or heterocyclic radical and in which the alkyl portion can also comprise an amino or acylamino substituent;

A can also be the residue

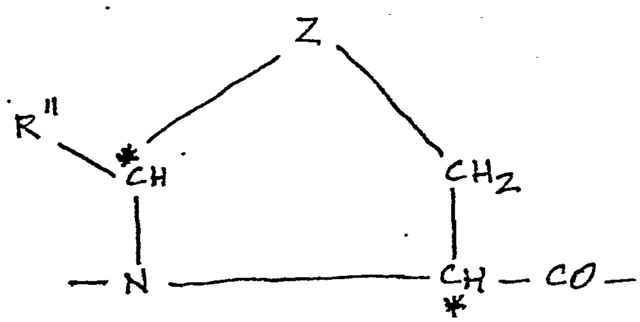

where Z is -S-, $-\overset{\overset{O}{\|}}{S}-$ or $-\overset{\overset{O}{\|}}{\underset{\underset{O}{\|}}{S}}-$,

and $R^{11}$ is hydroxy, alkyloxy, arylalkyleneoxy, aryloxy, acyloxy, carboxy, alkyloxycarbonyl, arylalkyleneoxycarbonyl, aryloxycarbonyl,

aminocarbonyl, mono or di-alkylaminocarbonyl, arylalkyleneamino-carbonyl, arylaminocarbonyl, amino, mono or di-alkylamino, aryl-alkyleneamino, arylamino, hydroxyalkylene, alkyloxyalkylene, arylalkyleneoxyalkylene, aryloxyalkylene, aminoalkylene, mono or di-alkylaminoalkylene, or arylaminoalkylene.

The asterisks indicate asymmetric carbon atoms (the carbons with the substituents $R^1$, $R^4$, $R^6$ are asymmetric when, respectively, $R^1$ is different from $R^2$, and $R^4$ and $R^6$ are other than hydrogen).

An alkyl group signifies a saturated or unsaturated hydrocarbon radical which can be linear, branched or cyclic and has a maximum of 6 carbon atoms (except where $R^1$ is alkyl, when this latter can have up to 20 carbon atoms), such as methyl, propyl, isopropyl, butyl, isobutyl, t-butyl, cyclohexyl, allyl, propargyl, cyclohexenyl and the like.

The alkylene group is of the same type, and also has a maximum of 6 carbon atoms.

An aryl group signifies either an unsubstituted phenyl radical, or a phenyl radical substituted with one or more substituents of the alkyl, hydroxy, alkyloxy, halogen, amino, mono or di-alkylamino, acylamino, carboxy, cyano, sulphonamide, aminoalkylene or haloalkylene type.

A heterocyclic radical signifies a saturated or unsaturated pentagonal or hexagonal cyclic group containing 1 or 2 heteroatoms, the remaining atoms being constituted by carbon and hydrogen, or their benzo derivatives; examples of these heterocyclic groups are the thienyl, furyl, pyrrolyl, imidazolyl, thiazolyl, oxazolyl, pyranyl, pyridyl, pyrimidyl, indolyl and like radicals, and the

corresponding partially or totally hydrogenated derivatives. An acyl group signifies a $R^{12}CO$ radical where $R^{12}$ is hydrogen, alkyl, aryl, arylalkylene, a heterocyclic radical, or an alkyl substituted with a heterocyclic radical.

The compounds corresponding to general formula I can form salts with numerous inorganic and organic bases when they contain carboxyl groups, and with mineral and organic acids when they contain basic groups (amino, mono or dialkylamino and the like). The salts according to the present invention comprise all the pharmaceutically acceptable salts.

These salts are represented for example by ammonium salts; by the salts of alkaline metals such as sodium, potassium or lithium; by the salts of alkaline earth metals such as calcium or magnesium; by the salts of organic bases such as dicyclohexylamine, diethanolamine, pyridine, N-methyl-D-glucamine; by the salts of hydrochloric, tartaric, acetic, ascorbic, oxalic, citric or like acids; and by the salts of amino acids such as glycine, lysine or arginine.

The compounds of formula I can exist in diastereoisomer form or in raceme mixture. All the optically active and optically inactive forms are included in the present invention. The compounds of formula I can be prepared by using the various methods described for preparing analogous products in USA patent No. 4,052,511 (N-carboxyalkanoyl derivatives of amino acids) and in European patent application No. 0,012,401 or in Nature, 288, 280 (1980) (N-carboxymethyl derivatives of dipeptides).

The starting substances of type $H-A-R^5$, where A and $R^5$ have the meaning indicated heretofore, are prepared as follows:

- when A is

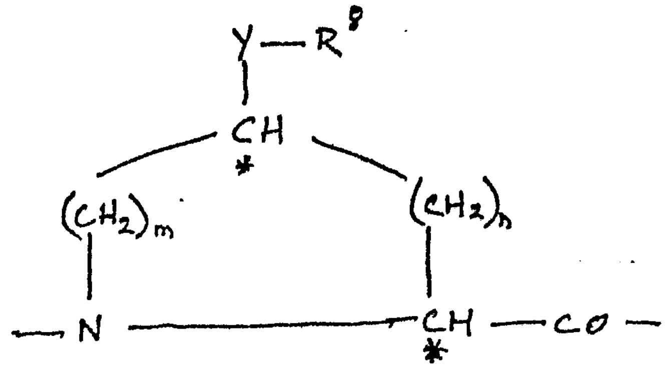

or

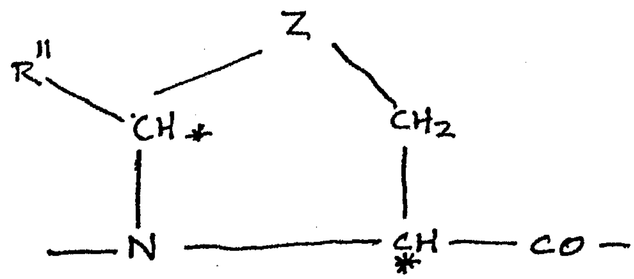

the methods used can be those cited in the copending Italian patent application of the present applicant No. 20,825 A/81, in German patent application No. 2,752,719 or in Dutch patent application No. 78 098806.

- when A is

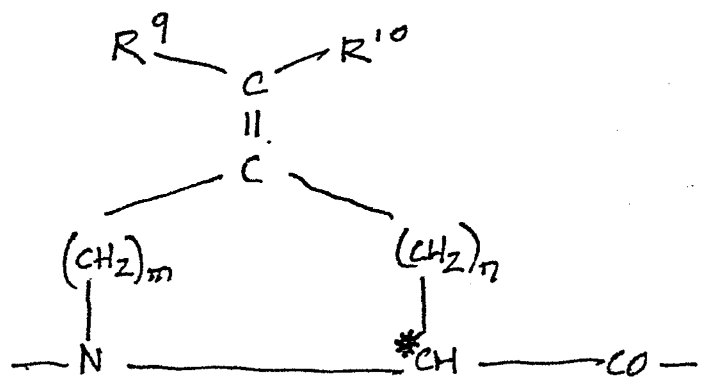

the starting substance can be prepared by the Wittig reaction (see for example Modern Synthetic Reactions II, Ed. W.A. Benjamin, Menlo Park, California 1971, p. 682-709), by reacting the ketone precursor of formula

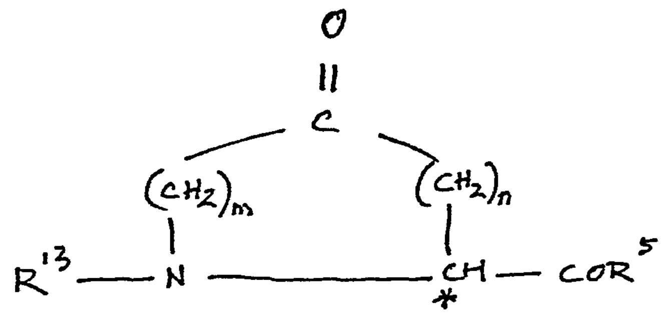

with a phosphorane of formula

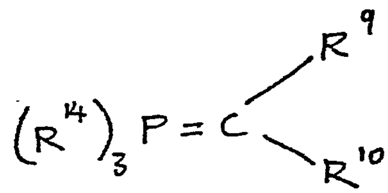

where m, n, $R^5$, $R^9$ and $R^{10}$ have the meaning indicated heretofore, $R^{13}$ is a conventional protector group which is removed after the Wittig reaction, and $R^{14}$ is alkyl or phenyl.

The products of formula I can also be obtained by alternative methods, such as that described in example 13.

The inhibition of the angiotensin conversion enzyme by the compounds of general formula I can be measured, in vitro, with the enzyme isolated from rabbit lungs by the method of Cushman and Cheing (Biochem. Pharmacol. 20,1637,(1971)), or by determining the variation caused by said compounds in the myotropic effect induced by angiotensin I and by bradykinin in the vas deferens of the rat (Magnan and Regoli, Can. J. Physyol. Pharmacol. 57, 417 (1979)).

It can be measured in vivo by determining the effects of the compounds of the present invention on the variations in the average arterial pressure induced by administering angiotensin I and bradykinin to anesthetised rats.

The subject matter and object of the invention will be more apparent

from reading the examples described hereinafter for illustrative purposes only and are in no way limitative of the invention.

EXAMPLE 1

1-(4-carboxybutyroyl)-allo-4-phenylthio-L-proline

1a) 1-tert.butoxycarbonyl-4-hydroxy-L-proline methyl ester

A solution of $CH_2N_2$ in ethyl ether is added drop by drop under an inert atmosphere to a stirred solution, cooled to $0^oC$, of 6.0 g (26 mmoles) of 1-(tert.butoxycarbonyl)-4-hydroxy-L-proline in 15 ml of ethyl ether, until a persistent yellow colouration is obtained. The solution is evaporated to dryness under vacuum to give 6.0 g of the required product (yield 94%).

$^1H$-NMR($CDCl_3$): $\delta$ 1.43 (9H, s, $(CH_3)_3C$), 2.16 (2H, m, $CH_2$-CH), 3.26-3.90 (5H, m, $CH_2N$ and $CH_3OCO$), 4.43 (2H, m, CHCOO and CH-OH), 5.50 (1H, s, OH).

Mass spectrum: m/e 245 ($M^+$), 190, 186, 172, 86, 68, 57, 41.

1b) 1-tert.butoxycarbonyl-4-tosyloxy-L-proline methyl ester

6.0 g (30 mmoles) of p-toluenesulphonylchloride dissolved in 8 ml of anhydrous pyridine are added to 6.0 g (24.5 mmoles) of 1-tert.butoxycarbonyl-4-hydroxy-L-proline methyl ester dissolved in 15.5 ml of anhydrous pyridine cooled to $-10^oC$. After stirring at $4^oC$ for 24 hours, the mixture is acidified with 150 ml of 2N HCl, then decanting the precipitate, which is taken up in 100 ml of ethyl acetate. The solution is extracted with a saturated aqueous solution of NaCl (2 x 50 ml). The organic phase is dried ($MgSO_4$) and then evaporated to dryness under vacuum.

6.2 g of the required product are obtained (yield 63%).

$^1$H-NMR($CDCl_3$): δ 1.4 (9H, s, $(CH_3)_3C$), 2.0-2.60 (5H, m, $CH_2$-$CH_2$ and $CH_3$-$C_6H_4$), 3.40-3.90 (5H, m, $CH_2N$ and $CH_3OCO$), 4.36 (1H, t, CHCOO), 5.10 (1H, m, CHO), 7.20-7.90 (4H, m, $C_6H_4$).

Mass spectrum: m/e $M^+$ absent, 343, 340, 284, 240, 227, 171, 68, 57.

1c) 1-tert.butoxycarbonyl-allo-4-phenylthio-L-proline methyl ester

660 mg (5 mmoles) of sodium thiophenate (freshly prepared by adding sodium hydride to a solution of thiophenol in tetrahydrofuran followed by evaporating the solvent under vacuum) are added under an inert atmosphere to 1.6 g (4 mmoles) of 1-tert.butoxycarbonyl-4-tosyloxy-L-proline methyl ester dissolved in 7.5 ml of anhydrous dimethylsulphoxide. After reacting at ambient temperature for 24 hours, 100 ml of a saturated aqueous solution of NaCl are added, and the mixture extracted with ethyl acetate (3 x 50 ml). The organic phase is dried ($Na_2SO_4$) and concentrated under vacuum. The residue is chromatographed in a silica gel column, eluting with petroleum ether-ethyl acetate (8:2). 750 mg of the required product are obtained (yield 55%).

$^1$H-NMR (CDCl): δ 1.40 (9H, s, $(CH_3)_3C$), 1.83-2.73 (2H, m, $CH_2$-CH), 3.16-4.53 (7H, m, $CH_2N$, $CH_3OCO$, CHCOO and CHS), 7.33 (5H, m, $C_6H_5$).

Mass spectrum: m/e 337 ($M^+$), 281, 278, 236, 178, 68, 57, 41.

1d) Allo-4-phenylthio-L-proline methyl ester hydrochloride

10 ml of methanol saturated with gaseous HCl at 0 °C are added

to 750 mg (2.2 mmoles) of 1-tert.butoxycarbonyl-allo-4-phenylthio-L-proline methyl ester dissolved in 1.0 ml of methanol. After 5 minutes, the solvent is evaporated under vacuum and the residue taken up in 15 ml of methanol, the solvent again being evaporated under vacuum.

570 mg of the required product are obtained (yield 95%).

$^1$H-NMR ($CDCl_3$): δ 1.83-3.0, (2H, m, $CH_2$-CH), 3.23-4.40 (6H, m, $CH_2N$, $CH_3OCO$ and CHCOO), 4.76 (1H, m, CHS) 7.33 (5H, m, $C_6H_5$), 7.33 (5H, m, $C_6H_5$), 10.20 (2H, s, $NH_2^+$).

1e) <u>1-(4-methoxycarbonylbutyroyl)-allo-4-phenylthio-L-proline methyl ester</u>

198 μl (1.42 mmoles) of triethylamine are added slowly under stirring and cooling (0°C) to a solution of 390 mg (1.42 mmoles) of allo-4-phenylthio-L-proline methyl ester hydrochloride and 207 mg (1.42 mmoles) of 4-methoxycarbonylbutyric acid in 1.5 ml of $CH_2Cl_2$. The mixture is left to come to ambient temperature for 10 minutes. To the resultant suspension, which is again cooled to 0°C, is added a solution of 293 mg (1.42 mmoles) of N,N'-dicyclohexylcarbodiimide in 1 ml of $CH_2Cl_2$, and the mixture is stirred at ambient temperature for 20 hours. After evaporating the solvent under vacuum, the residue is taken up in 25 ml of ethyl acetate and the suspension is filtered, washing the filtrate successively with 1N HCl, $H_2O$, 1% aqueous $NaHCO_3$, and finally $H_2O$.

The organic phase is dried ($MgSO_4$) and evaporated under vacuum to give 575 mg of product. This latter is further purified by chromatography in a silica gel column, eluting with ethyl

acetate-petroleum ether (70:30) to give 320 mg of the required product (yield 61%).

$^1H$ NMR ($CDCl_3$): δ 1.65-2.55 (8H, m, $(CH_2)_3$ and $\underline{CH_2}$-CH), 3.50-3.95 (9H, m, $COOCH_3CH_2N$, CHS), 4.50 (1H, t, CHCOO), 7.33 (5H, m, $C_6H_5$).

Mass spectrum: m/e 365 ($M^+$), 334, 306, 256, 178, 129, 110, 68.

1f) <u>1-(4-carboxybutyroyl)-allo-4-phenylthio-L-proline</u>

3.5 ml of 1N NaOH are added under stirring to 320 mg (0.875 mmoles) of 1-(4-methoxycarbonylbutyroyl)-allo-4-phenylthio-L-proline methyl ester suspended in 1.8 ml of cold methanol ($0^o$). The mixture is stirred at ambient temperature for 7 hours, then diluted with 15 ml of $H_2O$ and extracted with 10 ml of ethyl acetate. The aqueous phase is acidified to pH 2.5 with 1N HCl, saturated with NaCl and extracted with ethyl acetate (4 x 20 ml). The organic extract is dried ($MgSO_4$) and then evaporated to dryness under vacuum to give 280 mg of the required product (yield 94%).

$^1H$-NMR($CDCl_3$): δ 1.60-2.80 (8H, m, $(CH_2)_3$ and $\underline{CH_2}$-CH), 3.20-4.20 (3H, m, $CH_2N$ and CHS), 4.50 (1H, t, CHCOO), 7.35 (5H, m, $C_6H_5$), 11.06 (2h, s, COOH).

Mass spectrum: m/e 337 ($M^+$), 319, 293, 275, 260, 228, 178, 110, 68.

<u>EXAMPLE 2</u>

<u>1-(4-carboxybutyroyl)-allo-4-phenoxy-L-proline</u>

2a) <u>1-tert.butoxycarbonyl-allo-4-phenoxy-L-proline methyl ester</u>

650 mg (5.6 mmoles) of sodium phenate (freshly prepared by reacting phenol with sodium hydride in anhydrous tetrahydrofuran followed by evaporating the solvent under vacuum) are added under an inert atmosphere to 2.0 g (5 mmoles) of 1-(tert. butoxycarbonyl)-4-tosyloxy-L-proline methyl ester (example 1b) dissolved in 8 ml of dimethylsulphoxide. The mixture is heated to 60°C for 4 hours and then left overnight at ambient temperature. 20 ml of water are added, and the mixture is extracted with 50 ml of ethyl acetate. The organic extract is washed twice with 1% $NaHCO_3$ saturated with NaCl, dried ($Na_2SO_4$) and chromatographed in a silica gel column, eluting with petroleum ether-ethyl acetate (6:4). 700 mg of the required product are obtained (yield 43%).

$^1H$-NMR($CDCl_3$): δ 1.43 (9H, s, $(CH_3)_3C$), 2.43 (2H, m, $\underline{CH_2}$-CH), 3.4-3.93 (5H, m, $CH_2N$ and $CH_3OCO$), 4.40 (1H, m, CHCOO), 4.93 (1H, m, CHO), 6.66-7.6 (5H, m, $C_6H_5$).

Mass spectrum: m/e 321 ($M^+$), 262, 227, 220, 168, 112, 162, 68.

2b) Allo-4-phenoxy-L-proline methyl ester hydrochloride

700 mg (2.17 mmoles) of 1-tert.butoxycarbonyl-allo-4-phenoxy-L-proline methyl ester are used as the starting substance in the procedure of example 1d, to give 514 mg of the required product (yield 92%).

Mass spectrum: m/e 221 ($M^+$), 162, 127, 94, 68, 41.

2c) 1-(4-methoxycarbonylbutyroyl)-allo-4-phenoxy-L-proline methyl ester

The allo-4-phenylthio-L-proline methyl ester hydrochloride is replaced in the procedure of example 1e by allo-4-phenoxy-L-proline methyl ester hydrochloride, to give the required product.

2d) <u>1-(4-carboxybutyroyl)-allo-4-phenoxy-L-proline</u>

The required product is obtained by hydrolysis of 1-(4-methoxycarbonylbutyroyl)-allo-4-phenoxy-L-proline methyl ester under the conditions described in example 1f.

EXAMPLE 3

1-(4-carboxybutyroyl)-allo-4-methylthio-L-proline

3a) 1-tert.butoxycarbonyl-allo-4-methylthio-L-proline methyl ester

0.580 g (8.28 mmoles) of sodium methyl mercaptide are added under an inert atmosphere to 2.800 g (7.02 mmoles) of 1-tert.butoxycarbonyl-4-tosyloxy-L-proline methyl ester (example 1b) dissolved in 12 ml of anhydrous dimethylsulphoxide. After reacting at ambient temperature for 24 hours, 100 ml of a saturated aqueous solution of NaCl are added, and the mixture extracted with ethyl acetate (3 x 50 ml). The organic phase is dried ($Na_2SO_4$) and concentrated under vacuum. The residue is chromatographed in a silica gel column, eluting with petroleum ether-ethyl acetate (8:2). 0.931 g of the required product are obtained (yield 48.1%).

$^1$H-NMR($CDCl_3$): $\delta$ 1.42 (9H, s, $(CH_3)_3C$), 2.15 (3H, s, $CH_3S$), 2.20-2.80 (2H, m, $CH_2$-CH), 3.30 (2H, m, $CH_2N$), 3.78 (3H, s, $COOCH_3$), 3.97 (1H, m, CHS), 4.32 (1H, m, CHCOO).

Mass spectrum: m/e 275 ($M^+$), 219, 216, 161, 116, 68, 57, 47.

3b) Allo-4-methylthio-L-proline methyl ester hydrochloride

0.931 g (3.38 mmoles) of 1-tert.butoxycarbonyl-allo-4-methylthio-L-proline methyl ester dissolved in 2 ml of methanol are used as the starting substance in the procedure of example 1d, to give 0.64 g of the required product (yield 90.6%).

0115091

3c) 1-(4-carboxybutyroyl)-allo-4-methylthio-L-proline methyl ester

The allo-4-phenylthio-L-proline methyl ester hydrochloride is replaced in the procedure of example 1e by allo-4-methylthio-L-proline methyl ester hydrochloride, to give the required product.

3d) 1-(4-carboxybutyroyl)-allo-4-methylthio-L-proline

The required product is obtained by hydrolysis of 1-(4-methoxycarbonylbutyroyl)-allo-4-methylthio-L-proline methyl ester under the conditions described in Example 1f.

EXAMPLE 4

1-(4-carboxybutyroyl)-allo-4-benzylthio-L-proline

4a) 1-(tert.butoxycarbonyl)-allo-4-benzylthio-L-proline methyl ester

0.292 g (2 mmoles) of sodium benzylmercaptide (freshly prepared by reacting benzylmercaptide with sodium hydride in anhydrous tetrahydrofuran followed by evaporating the solvent under vacuum) are added under an inert atmosphere to 0.350 g (0.88 mmoles) of tert.butoxycarbonyl-4-tosyloxy-L-proline methyl ester (example 1b) dissolved in 5 ml of anhydrous dimethylsulphoxide. The mixture is heated to 60°C for 4 hours and then left overnight at ambient temperature. 20 ml of a saturated aqueous solution of NaCl are added, and the mixture extracted with ethyl acetate (3 x 50 ml). The organic phase is dried over $Na_2SO_4$ and concentrated under vacuum. The residue is chromatographed in a silica gel column, eluting with petroleum ether-ethyl acetate (8:2). 0.157 g of the required product are obtained (yield 50%).

4b) Allo-4-benzylthio-L-proline methyl ester hydrochloride

0.157 g (0.5 mmoles) of 1-tert.butoxycarbonyl-allo-4-benzylthio-L-proline methyl ester dissolved in 2 ml of methanol are used as the starting substance, together with 5 ml of methanol saturated with gaseous HCl, in the procedure of example 1d to give 0.116 g of the required product (yield 90%).

4c) 1-(4-methoxycarbonylbutyroyl)-allo-4-benzylthio-L-proline methyl ester

The allo-4-phenylthio-L-proline methyl ester hydrochloride is replaced in the procedure of example 1e by allo-4-benzylthio-L-proline methyl ester hydrochloride, to give the required product.

4d) 1-(4-carboxybutyroyl)-allo-4-benzylthio-L-proline

The required product is obtained by hydrolysis of 1-(4-methoxycarbonyl)-allo-4-benzylthio-L-proline methyl ester under the conditions described in example 1f.

EXAMPLE 5

1-(4-carboxybutyroyl)-4-benzoxy-L-proline

5a) 1-tert.butoxycarbonyl-4-benzoxy-L-proline

100 mg (4.32 mequiv) of sodium and, after 30 minutes, 0.248 ml (2.16 mmoles) of benzyl chloride are added under an inert atmosphere to a stirred solution, cooled to -78°C, of 500 mg (2.16 mmoles) of 1-(tert.butoxycarbonyl)-4-hydroxy-L-proline in 20 ml of liquid ammonia. After 5 hours of reaction, the mixture is left to come to ambient temperature until complete evaporation of the ammonia, is then taken up in 20 ml of $H_2O$, and extracted with 10 ml of $CH_2Cl_2$.

The aqueous phase is acidified to pH 2.5 with 1N HCl, and extracted with $CH_2Cl_2$ (3 x 20 ml). The extract is dried ($MgSO_4$) and evaporated to dryness under vacuum. 500 mg of the required product are obtained (yield 72%).

$^1$H-NMR($CDCl_3$): δ 1.43 (9H, s, $(CH_3)_3C$), 2.30 (2H, m, $\underline{CH_2}$-CH), 3.66 (2H, m, $CH_2N$), 4.33 (2H, m, CHCOO and CHO), 4.53 (2H, s, $CH_2$-$C_6H_5$), 7.38 (5H, s, $C_6H_5$), 9.25 (1H, s, COOH).

Mass spectrum: m/e $M^+$ absent, 277, 266, 220, 176, 130, 91, 68, 57.

5b) <u>4-benzoxy-L-proline methyl ester hydrochloride</u>

250 mg (0.78 mmoles) of 1-tert.butoxycarbonyl-4-benzoxy-L-proline are used as the starting substance in the procedure of example 1d, to give 200 mg of the required product (yield 94%).

$^1$H-NMR($CDCl_3$): δ 2.46 (2H, m, $\underline{CH_2}$-CH), 3.23-4.10 (5H, m, $CH_2N$ and $CH_3OCO$), 4.10-5.0 (4H, m, CHCOO, CHO and $\underline{CH_2}$-$C_6H_5$), 7.40 (5H, s, $C_6H_5$), 9.60 (2H, s, $NH_2^+$).

Mass spectrum: m/e 235 ($M^+$), 176, 144, 129, 91, 70, 68.

5c) <u>1-(4-methoxycarbonylbutyroyl)-4-benzoxy-L-proline methyl ester</u>

The allo-4-phenylthio-L-proline methyl ester hydrochloride is replaced in the procedure of example 1e by 4-benzoxy-L-proline methyl ester hydrochloride to give the required product.

5d) <u>1-(4-carboxybutyroyl)-4-benzoxy-L-proline</u>

The required product is obtained by hydrolysis of 1-(4-methoxy-carbonylbutyroyl)-4-benzoxy-L-proline methyl ester under the conditions described in example 1f.

EXAMPLE 6

1-(4-carboxybutyroyl)-4-allyloxy-L-proline

6a) 1-tert.butoxycarbonyl-4-allyloxy-L-proline

200 mg (8.64 mequiv) of sodium and, after 60 minutes, 0.374 ml (4.32 mmoles) of allyl bromide are added under an inert atmosphere to a stirred and cooled (-78°C) solution of 1.0 g (4.32 mmoles) of 1-(tert.butoxycarbonyl)-4-hydroxy-L-proline in 40 ml of liquid ammonia. After 4 hours of reaction, the mixture is allowed to come to ambient temperature until the ammonia has completely evaporated, is then taken up in 40 ml of $H_2O$ and extracted with 20 ml of $CH_2Cl_2$. The aqueous phase is acidified to pH 2.5 with 1N HCl, and extracted with $CH_2Cl_2$ (3x40 ml).

The extract is dried ($MgSO_4$) and evaporated to dryness under vacuum. 935 mg of the required product are obtained (yield 80%).

$^1$H-NMR($CDCl_3$): δ 1.46 (9H, s, $(CH_3)_3C$), 2.30 (2H, m, $CH_2$-CHCOO), 3.62 (2H, s, $CH_2N$), 3.83-4.73 (4H, m, $OCH_2$, CHCOO and CHO), 5.0-6.33 (3H, m, $CH=CH_2$), 10.43 (1H, s, COOH).

Mass spectrum: m/e ($M^+$) absent, 226, 198, 170, 126, 84, 68, 57, 41.

6b) 4-allyloxy-L-proline methyl ester hydrochloride

557 mg (2.05 mmoles) of 1-tert.butoxycarbonyl-4-allyloxy-L-proline are used as the starting substance, together with 20 ml of methanol saturated with gaseous HCl, in the procedure of example 1d to give 430 mg of the required product (yield 94%).

$^1$H-NMR($CDCl_3$): δ 2.46 (3H, m, $CH_2$-CHCOO), 3.04-4.83 (9H, m,

$CH_2N$, $CH_3OCO$, $CH_2O$, CHCOO and CHO), 5.0-6.46 (3H, m, $CH=CH_2$), 10.10 (2H, s, $N^+H_2$).

Mass spectrum: m/e 185 ($M^+$), 129, 126, 84, 68, 41.

6c) 1-(4-methoxycarbonylbutyroyl)-4-allyloxy-L-proline methyl ester

The allo-4-phenylthio-L-proline methyl ester hydrochloride is replaced in the procedure of example 1e by 4-allyloxy-L-proline methyl ester hydrochloride, to give the required product.

6d) 1-(4-carboxybutyroyl)-4-allyloxy-L-proline

The required product is obtained by hydrolysis of 1-(4-methoxycarbonylbutyroyl)-4-allyloxy-L-proline methyl ester under the conditions described in example 1f.

EXAMPLE 7

N-(1-carboxy-3-phenylpropyl-L-alanyl-allo-4-phenylthio-L-proline

7a) Tert.butoxycarbonyl-L-alanyl-allo-4-phenylthio-L-proline methyl ester

0.7 ml of triethylamine, 0.945 g (5 mmoles) of tert.butoxycarbonyl-L-alanine and 1.030 g of N,N'-dicyclohexylcarbodiimide are added to a solution, cooled to 0°C, of 1.365 g (5 mmoles) of allo-4-phenylthio-L-proline methyl ester hydrochloride (example 1d) dissolved in 9 ml of methylene chloride. The mixture is stirred for 24 hours at ambient temperature. After evaporating the solvent under vacuum, the residue is taken up in 30 ml of ethyl acetate and the suspension filtered, washing the filtrate successively with 1N HCl, $H_2O$, 1% aqueous $NaHCO_3$ and finally $H_2O$. The organic phase is dried ($Na_2SO_4$), concentrated under vacuum and chromatographed in a silica gel column, eluting with ethyl acetate-petroleum ether (70:30) to give 1.700 g of the

required product (yield 83.2%).

$^1H$-NMR($CDCl_3$): δ 1.20-1.50 (12H, m, $(CH_3)_3C$ and $CH_3$-CH), 2.20-2.80 (2H, m, $CH_2$-CH), 3.40-3.90 (5H, m, $CH_2N$, $COOCH_3$), 4.04-4.70 (3H, m, CHS, CHCOO, CH-NH), 5.56 (1H, d, NH), 7.36 (5H, m, $C_6H_5$).

Mass spectrum: m/e 408 ($M^+$), 352, 335, 299, 276, 178, 167, 68.

7b) Tert.butoxycarbonyl-L-alanyl-allo-4-phenylthio-L-proline

1.70 g (4.16 mmoles) of tert.butoxycarbonyl-L-alanyl-allo-4-phenylthio-L-proline methyl ester are hydrolysed with a mixture of 10 ml of methanol and 15 ml of 1N NaOH for 2 hours at ambient temperature. The solution is acidified with cold 6N HCl and extracted with 150 ml of ethyl acetate. The organic phase, dried over $Na_2SO_4$, is concentrated under vacuum to give 1.60 g of the required product (yield 97.5%).

7c) L-alanyl-allo-4-phenylthio-L-proline hydrochloride

1.60 g (4.06 mmoles) of tert.butoxycarbonyl-L-alanyl-allo-4-phenylthio-L-proline are dissolved in 30 ml of ethyl acetate saturated with gaseous HCl at 0°C. The solution is stirred at ambient temperature for 15 minutes and evaporated under vacuum. The residue is taken up several times in ethyl acetate and the solvent evaporated in order to remove HCl traces. 1.20 g of the required product are obtained (yield 89.4%).

7d) N-(1-carboxy-3-phenylpropyl)-L-alanyl-allo-4-phenylthio-L-proline

1.20 g (3.63 mmoles) of L-alanyl-allo-4-phenylthio-L-proline hydrochloride and 3.56 g (20 mmoles) of benzylpyruvic acid

are dissolved in a mixture of 24 ml of ethanol and 20 ml of $H_2O$, and the pH is adjusted to 7.0 with 6N NaOH. 1.00 g (15.2 mmoles) of sodium cyanoborohydride are added to the solution, which is then stirred at ambient temperature for 24 hours. 50 ml of Dowex W 50 resin of $H^+$ form are added to the solution, and the mixture after stirring under vacuum in order to decompose the residual hydride is fed to a chromatograph column containing 40 ml of the same resin. It is eluted with ethanol $-H_2O$ (1:1) until neutral, and the product is eluted with a solution of 2% pyridine in ethanol $-H_2O$ (1:1).

The fractions rich in the product are combined, concentrated under vacuum and further purified by chromatography over a Dowex AG-1 resin of $HCOO^-$ form, eluting with a linear gradient of HCOOH (0-1N) in ethanol $-H_2O$ (1:1). 1.045 g of the required product are recovered by lyophilisation (yield 63.1%).

$^1$H-NMR($C_5D_5N$): δ 1.40 (3H, d, $CH_3$), 1.85-3.15 (6H, m, $(CH_2)_2$ and <u>CH</u>$_2$-CH), 3.30-5.0 (6H, m, $CH_2N$, CHS, CHCOO, <u>CH</u>-NH-<u>CH</u>), 7.25 (10H, m, $C_6H_5$), 10.80 (3H, widened s, COOH, NH).

Mass spectrum: $M^+$ absent, m/e 438, 420, 392, 334, 275, 206, 178, 110, 68.

<u>EXAMPLE 8</u>

<u>N-(1-carboxy-3-phenylpropyl)-L-alanyl-allo-4-phenoxy-L-proline</u>

8a) <u>Tert.butoxycarbonyl-L-alanyl-allo-4-phenoxy-L-proline methyl ester</u>

By treating 0.866 g (3.37 mmoles) of allo-4-phenoxy-L-proline methyl ester hydrochloride (example 2b) with 0.650 g (3.43

mmoles) of tert.butoxycarbonyl-L-alanine, 0.5 ml of triethylamine and 0.700 g (3.40 mmoles) of N, N'-dicyclohexylcarbodiimide under the conditions described in example 7a, 0.991 g of the required product are obtained (yield 74.8%).

$^1$N-NMR($CDCl_3$): δ 1.25-2.45 (12H, s, $\underline{CH_3}$-CH and $(CH_3)_3C$), 2.50 (2H, m, $\underline{CH_2}$-CH), 3.70 (3H, s, $COOCH_3$), 3.90-5.0 (5H, m, $CH_2N$, CHCOO, CHO, $\underline{CH}$-NH), 6.80-7.50 (5H, m, $C_6H_5$).

Mass spectrum: m/e 392 ($M^+$), 336, 319, 242, 220, 162, 144, 68.

8b) Tert.butoxycarbonyl-L-alanyl-allo-4-phenoxy-L-proline

0.670 g (1.71) mmoles of tert.butoxycarbonyl-L-alanyl-allo-4-phenoxy-L-proline methyl ester are hydrolysed with a mixture of 5 ml of methanol and 8 ml of 1N NaOH under the conditions described in example 7b, to give 0.605 g of the required product (yield 93.6%).

Mass spectrum: m/e 378 ($M^+$), 334, 322, 305, 260, 166, 162, 68.

8c) L-alanyl-allo-4-phenoxy-L-proline hydrochloride

0.605 g (1.60 mmoles) of tert.butoxycarbonyl-L-alanyl-allo-4-phenoxy-L-proline are used as the starting substance in the procedure of example 7c, to give 0.472 g of the required product (yield 93.9%).

Mass spectrum: m/e 278 ($M^+$), 260, 167, 166, 162, 68, 36, 38.

8d) N-(1-carboxy-3-phenylpropyl)-L-alanyl-allo-4-phenoxy-L-proline

0.472 g (1.50 mmoles) of L-alanyl-allo-4-phenoxy-L-proline hydrochloride and 1.340 g (7.53 mmoles) of benzylpyruvic acid are dissolved in a mixture of 10 ml of ethanol and 8 ml of

$H_2O$, and after adjusting the pH to 7.0 (6N NaOH), 0.400 g (6.35 mmoles) of sodium cyanoborohydride are added. By proceeding in the manner described in example 7d, 0.370 g of the required product are obtained (yield 56.1%).

$^1H$-NMR($CD_3OD$): δ 1.50 (3H, m, $CH_3$), 2.0-3.0 (6H, m, $(CH_2)_2$, $CH_2CH$), 3.75 (2H, m, $CH_2N$), 4.0-4.8 (3H, m, CH-NH-CH, CHCOO), 6.80-7.45 (10H, m, $C_6H_5$).

Mass spectrum: $M^+$ absent, m/e 422, 404, 376, 318, 300, 206, 162, 68.

EXAMPLE 9

N-(1-carboxy-3-phenylpropyl)-L-alanyl-allo-4-methylthio-L-proline

9a) Tert.butoxycarbonyl-L-alanyl-allo-4-methylthio-L-proline methyl ester

0.644 g (3.06 mmoles) of allo-4-methylthio-L-proline methyl ester hydrochloride (example 3b) are treated with 0.550 g (3.1 mmoles) of tert.butoxycarbonyl-L-alanine, 0.43 ml of triethylamine and 0.630 g (3.06 mmoles) of N,N'-dicyclohexylcarbodiimide under the conditions described in example 7a, to give 0.940 g of the required product (yield 88.8%).

$^1H$-NMR($CDCl_3$): δ 1.22-1.60 (12H, m, $CH_3$-CH and $(CH_3)_3C$), 2.20 (3H, s, $CH_3S$), 2.15-2.20 (2H, m, $CH_2$-CH), 3.40 (2H, m, $CH_2$-N), 3.70 (3H, s, $COOCH_3$), 4.0-4.80 (3H, m, CHS, CHCOO, CH, NH), 5.70 (1H, d, NH).

9b) Tert.butoxycarbonyl-L-alanyl-allo-4-methylthio-L-proline

0.940 g (2.72 mmoles) of tert.butoxycarbonyl-L-alanyl-allo-4-methylthio-L-proline methyl ester are hydrolysed with a mixture of 7 ml of methanol and 11 ml of 1N NaOH under the

conditions described in example 7b, to give 0.890 g of the required product (yield 98.7%).

Mass spectrum: m/e 332 ($M^+$), 288, 285, 276, 229, 214, 116, 68.

9c) L-alanyl-allo-4-methylthio-L-proline hydrochloride

0.890 g (2.69 mmoles) of tert.butoxycarbonyl-L-alanyl-allo-4-methylthio-L-proline are used as the starting substance in the procedure of example 7c, to give 0.702 g of the required product (yield 97.7%).

Mass spectrum: m/e 232 ($M^+$), 214, 168, 167, 166, 116, 68, 36, 38.

9d) N-(1-carboxy-3-phenylpropyl)-L-alanyl-allo-4-methylthio-L-proline

0.702 g (2.62 mmoles) of L-alanyl-allo-4-methylthio-L-proline hydrochloride and 2.70 g (15.16 mmoles) of benzylpyruvic acid are dissolved in a mixture of 18 ml of methanol and 15 ml of $H_2O$, the pH is adjusted to 7.0, and 0.750 g (11.90 mmoles) of sodium cyanoborohydride are added. Following the procedure described in example 7d, 0.658 g of the required product are obtained (yield 63.7%).

$^1H$-NMR($CD_3OD$): δ 1.60 (3H, m, $CH_3$-CH), 2.02-2.40 (5H, m, $CH_2$-$CH_2$-$C_6H_5$, $CH_3S$), 2.60-3.05 (4H, m, $CH_2$-$C_6H_5$, $CH_2$-CH), 3.70 (2H, m, $CH_2$, m, $CH_2N$), 4.05-4.80 (4H, m, CH-NH-CH-CHCOO, CHS), 7.35 (5H, s, $C_6H_5$).

Mass spectrum: $M^+$ absent, m/e 376, 329, 272, 254, 206, 116, 91, 68.

EXAMPLE 10

N-(1-carboxy-3-phenylpropyl)-L-alanyl-allo-4-benzylthio-L-proline

10a) Tert.butoxycarbonyl-L-alanyl-allo-4-benzylthio-L-proline

The allo-4-phenylthio-L-proline methyl ester hydrochloride is replaced in the procedure of example 7a by allo-4-benzylthio-L-proline methyl ester hydrochloride (example 4b), to give the required product.

10b) Tert.butoxycarbonyl-L-alanyl-allo-4-benzylthio-L-proline

The required product is obtained by basic hydrolysis of tert. butoxycarbonyl-L-alanyl-allo-4-benzylthio-L-proline methyl ester by the procedure described in example 7b.

10c) L-alanyl-allo-4-benzylthio-L-proline hydrochloride

Tert.butoxycarbonyl-L-alanyl-allo-4-benzylthio-L-proline is used as the starting substance in the procedure of example 7c, to give the required product.

10d) N-(1-carboxy-3-phenylpropyl)-L-alanyl-allo-4-benzylthio-L-proline

The L-alanyl-allo-4-phenylthio-L-proline hydrochloride is replaced in the procedure of example 7d by L-alanyl-allo-4-benzylthio-L-proline hydrochloride, to give the required product.

EXAMPLE 11

N-(1-carboxy-3-phenylpropyl)-L-alanyl-4-benzoxy-L-proline

11a) Tert.butoxycarbonyl-L-alanyl-4-benzoxy-L-proline methyl ester

The allo-4-phenylthio-L-proline methyl ester hydrochloride is replaced in the procedure of example 7a by 4-benzoxy-L-proline methyl ester hydrochloride (example 5b), to give the required product.

11b) Tert.butoxycarbonyl-L-alanyl-4-benzoxy-L-proline

The required product is obtained by the basic hydrolysis of tert.butoxycarbonyl-L-alanyl-4-benzoxy-L-proline methyl ester by the procedure described in example 7b.

11c) L-alanyl-4-benzoxy-L-proline hydrochloride

Tert.butoxycarbonyl-L-alanyl-4-benzoxy-L-proline is used as the starting substance in the procedure of example 7c, to give the required product.

11d) N-(1-carboxy-3-phenylpropyl)-L-alanyl-4-benzoxy-L-proline

The L-alanyl-allo-4-phenylthio-L-proline hydrochloride is replaced in the procedure of example 7d by L-alanyl-4-benzoxy-L-proline hydrochloride, to give the required product.

EXAMPLE 12

N-(1-carboxy-3-phenylpropyl)-L-alanyl-4-allyloxy-L-proline

12a) Tert.butoxycarbonyl-L-alanyl-4-allyloxy-L-proline methyl ester

The allo-4-phenylthio-L-proline methyl ester is replaced in the procedure of example 7a by 4-allyloxy-L-proline methyl ester (example 6b), to give the required product.

12b) Tert.butoxycarbonyl-L-alanyl-4-allyloxy-L-proline

The required product is obtained by basic hydrolysis of tert.butoxycarbonyl-L-alanyl-4-allyloxy-L-proline methyl ester by the procedure described in example 7b.

12c) L-alanyl-4-allyloxy-L-proline hydrochloride

Tert.butoxycarbonyl-L-alanyl-4-allyloxy-L-proline is used as the starting substance in the procedure of example 7c to give the required product.

12d) N-(1-carboxy-3-phenylpropyl)-L-alanyl-4-allyloxy-L-proline

The L-alanyl-allo-4-phenylthio-L-proline hydrochloride is

replaced in the procedure of example 7d by L-alanyl-4-allyloxy-L-proline hydrochloride, to give the required product.

EXAMPLE 13

N-(1-carboxy-3-phenylpropyl)-L-alanyl-4-benzylidene-L-proline

13a) Tert.butoxycarbonyl-L-alanyl-4-keto-L-proline

A solution of 1.75 g (16.08 mmoles) of $Na_2CO_3$ in 30 ml of $H_2O$ followed by a suspension of 3.51 g (12.38 mmoles) of tert. butoxycarbonyl-L-alanine-N-hydroxysuccinimide ester in 30 ml of dioxane are added to a solution, cooled to 0°C, of 1.58 g (8.04 mmoles) of 4-keto-L-proline hydrobromide (prepared in accordance with J. Am. Chem. Soc. 79 185 (1957)) in 10 ml of dioxane -$H_2O$ (1:1). The mixture is kept vigorously stirred at 0°C for 24 hours, then alkalised to pH 10 with 2N NaOH and extracted with AcOet (6 x 30 ml). The aqueous phase is acidified to pH 2.5 with 6N HCl and extracted with AcOet (6 x 30 ml). The organic phase is filtered, dried ($MgSO_4$) and evaporated to dryness under vacuum to give 2.3 g of the required product (yield 92%).

$^1$H-NMR($CDCl_3$): $\delta$ 1.20-1.50 (12H, m, $(CH_3)_3C$ and $CH_3$), 2.77 (2H, m, $CH_2$-CH), 3.80-5.0 (4H, m, $CH_2N$, CHCOO, CH, NH).

Mass spectrum: m/e 300 ($M^+$), 244, 227, 182, 144, 88, 84, 57, 44.

13b) Tert.butoxycarbonyl-L-alanyl-4-benzylidene-L-proline

6.28 g (16.12 mmoles) of benzyltriphenylphosphonium chloride and 32 ml of anhydrous tetrahydrofuran are added to the mixture obtained by dissolving under anhydrous conditions 0.633 g (16.12 mequiv) of potassium in 15 ml of anhydrous

tert.butanol (2 hours at 50°C) after this latter mixture has been cooled to ambient temperature, the resultant mixture then being stirred for 2 hours. A solution of 2.0 g (6.45 mmoles) of tert.butoxycarbonyl-L-alanyl-4-keto-L-proline in 32 ml of anhydrous tetrahydrofuran are added to the resultant red suspension, and the mixture is stirred at 50°C under an inert atmosphere for 24 hours. After cooling, and then evaporating the solvent, the residue is taken up in 50 ml of ethanol -$H_2O$ (1:1), and the solution is fed to a chromatograph column containing 70 ml of DOWEX AG-1 resin in $HCOO^-$ form. After eluting with ethanol-$H_2O$ (1:1) until ultraviolet-absorbing material disappears in the eluate, the product is eluted with a linear gradient of HCOOH (0-0.5M) in ethanol-$H_2O$ (1:1). 1.82 g of the required product are recovered by concentration (yield 73.6%).

$^1H$-NMR($CDCl_3$): $\delta$ 1.20-1.50 (12H, m, $(CH_3)_3C$ and $CH_3$), 3.1 (2H, m, $\underline{CH_2}$-CH), 4.25-5.0 (4H, m, $CH_2N$, CHCOO, $\underline{CH}$, NH), 5.50 (1H, d, NH), 6.50 (1H, widened s, CH=), 7.25 (5H, m, $C_6H_5$).

Mass spectrum: m/e 374 ($M^+$), 318, 301, 256, 202, 158, 144, 88, 57.

13c) <u>L-alanyl-4-benzylidene-L-proline formate</u>

1.32 g (3.44 mmoles) of tert.butoxycarbonyl-L-alanyl-4-benzylidene-L-proline are dissolved in 20 ml of 98% formic acid and kept stirring at ambient temperature for 4 hours. The mixture is evaporated to dryness under vacuum, the residue is taken up in ether, and the mixture again evaporated to dryness to give 1.12 g of the required product (yield 100%).

Mass spectrum: $M^+$ absent, m/e 256, 241, 239, 158, 156, 129, 91, 44.

13d) N-(1-carboxy-3-phenylpropyl)-L-alanyl-4-benzylidene-L-proline

0.240 g (0.75 mmoles) of L-alanyl-4-benzylidene-L-proline formate and 1.4 g (7.87 mmoles) of benzylpyruvic acid are dissolved in 5 ml of $H_2O$ and the pH adjusted to 7.0 with 6N NaOH.

0.280 g (4.45 mmoles) of sodium cyanoborohydride are added to the solution, and the mixture is stirred at ambient temperature overnight. 25 ml of DOWEX W 50 resin in $H^+$ form are added to the solution, and the mixture, after stirring (2 hours) in order to decompose the hydride residue, is fed to a chromatograph column containing a further 25 ml of the same resin. It is eluted with ethanol-$H_2O$ (1:1) until neutral, after which the product is eluted with a 2% solution of pyridine in ethanol-$H_2O$ (1:1). The product-rich fractions are combined and concentrated under vacuum. The residue is further purified by chromatography on DOWEX AG-1 resin in $HCOO^-$ form, eluting with ethanol-$H_2O$ (1:1) and then with a linear gradient of formic acid (0-1N) in ethanol-$H_2O$ (1:1). 80 mg of the required product are recovered by concentration under vacuum followed by lyophilisation (yield 24.5%).

$^1H$-NMR($CH_3COOD$): $\delta$ 1.60 (3H, m, $CH_3$), 2.0-3.60 (6H, m, $(CH_2)_2$, $\underline{CH_2}$-CH), 3.75-5.15 (H, m, $CH_2$, CHCOO, $\underline{CH}$-NH-$\underline{CH}$), 6.70 (1H, widened s, CH=), 7.35 (10H, m, $C_6H_5$).

Mass spectrum: $M^+$ absent, m/e 418, 314, 296, 206, 158, 156, 44.

EXAMPLE 14

3-(4-carboxybutyroyl)-2-hydroxymethyl-L-4-thiazolidinecarboxylic acid

14a) 2-hydroxymethyl-L-4-thiazolidinecarboxylic acid

8.78 g (50 mmoles) of L-cysteine hydrochloride monohydrate are added to 25 ml of an aqueous solution of 3.0 g (50 mmoles) of glycolaldehyde, and the mixture is stirred for 2 hours at ambient temperature. After adjusting the pH to 6.0 with 2N NaOH and having added 25 ml of methanol, the precipitate is collected by filtration, and dried. 7.1 g of the required product are obtained (yield 87%).

$^1H$-NMR($D_2O$): δ 4.00 (2H, m, $CH_2S$), 4.43 (2H, m, $CH_2O$), 5.53 (2H, m, CHS and CHCOO).

14b) 2-hydroxymethyl-L-4-thiazolidinecarboxylic acid methyl ester

A flow of gaseous HCl is bubbled for 2 hours through a suspension of 2.5 g (15.3 mmoles) of 2-hydroxymethyl-L-4-thiazolidinecarboxylic acid in 100 ml of anhydrous methanol, cooled with an ice bath, after which the mixture is stirred overnight at +4°C.

After removing the solvent under vacuum, the residue is suspended in 30 ml of a saturated $NaHCO_3$ solution, and extracted with ethyl ether (3 x 100).

The ether phase is dried ($MgSO_4$), and then evaporated to dryness under vacuum to give 1.8 g of the required product (yield 67%).

Mass spectrum: m/e 177 ($M^+$), 159, 146, 118, 100, 86, 59.

14c) 3-(4-carboxybutyroyl)-2-hydroxymethyl-L-4-thiazolidinecarboxylic acid

0.547 g (4.8 mmoles) of glutaric anhydride are added under nitrogen to 0.85 g (4.8 mmoles) of 2-hydroxymethyl-L-4-thiazolidinecarboxylic acid methyl ester dissolved in 10 ml of glacial acetic acid and cooled to +5°C, and the mixture is stirred at ambient temperature for 2 days. After removing the acetic acid under vacuum, the residue is taken up in 10 ml of water, the mixture adjusted to pH 7.5 with 2N NaOH, and the solution extracted with ethyl ether (2 x 50). The aqueous phase is adjusted to pH 5.0 with HCl (1:1) and extracted with ethyl ether (2 x 100). This ether phase then being dried ($MgSO_4$) and the solvent evaporated under vacuum to give 0.62 g of 3-(4-carboxybutyroyl)-2-hydroxymethyl-L-4-thiazolidinecarboxylic acid methyl ester (yield 45%). The product is dissolved in 5 ml of ethanol, 4.5 ml of 1N NaOH are added, and the mixture stirred for 3 hours at ambient temperature. After removing the methanol under vacuum, the residue is extracted with ethyl ether (2 x 50). The aqueous phase is acidified to pH 5.0 with HCl (1:1), and extracted with ethyl ether (3 x 50), the product obtained from this ether phase by drying ($MgSO_4$) and evaporating the solvent under vacuum being further purified by chromatography in a silica gel column, eluting with acetonitrile-water (90:10). 0.35 g of the required product are obtained (overall yield 27%).

$^1$H-NMR ($D_2O$): $\delta$ 2.00 (2H, m, $CH_2$-$\underline{CH_2}$), 2.20-2.80 (4H, m, $CH_2$CON, $CH_2$COO), 3.50 (2H, m, $\underline{CH_2}$-CH), 3.90 (2H, m, $CH_2$O),

5.50 (1H, m, CHS).

EXAMPLE 15

N-(1-carboxy-3-phenylpropyl)-L-alanyl-2-hydroxymethyl-L-4-thiazolidine-carboxylic acid

15a) Tert.butoxycarbonyl-L-alanyl-2-hydroxymethyl-L-4-thiazolidinecarboxylic acid methyl ester

1.001 g (5.35 mmoles) of tert.butoxycarbonyl-L-alanine and 1.10 g of N, N'-dicyclohexylcarbodiimide are added to a solution, cooled to 0°C, of 0.95 g (5.35 mmoles) of 2-hydroxymethyl-L-4-thiazolidinecarboxylic acid methyl ester (example 14b) in 10 ml of methylene chloride. The mixture is stirred for 24 hours at ambient temperature. After evaporating the solvent under vacuum, the residue is taken up in 30 ml of ethyl acetate and the suspension filtered, washing the filtrate successively with 1N HCl, $H_2O$, and 1% aqueous $NaHCO_3$, to obtain 1.49 g of the required product (yield 80%).

15b) Tert.butoxycarbonyl-L-alanyl-2-hydroxymethyl-L-4-thiazolidinecarboxylic acid

1.49 (4.27 mmoles) of tert.butoxycarbonyl-L-alanyl-2-hydroxymethyl-L-4-thiazolidinecarboxylic acid methyl ester are hydrolysed by stirring at ambient temperature for 2 hours with a mixture of 10 ml of methanol and 10 ml of 1N NaOH.

After removing the methanol under vacuum, the solution is acidified with cold 6N HCl and extracted with diethylacetate (3 x 50). The organic phase is dried ($MgSO_4$) and concentrated under vacuum to give 1.21 g of the required product (yield 85%).

15c) L-alanyl-2-hydroxymethyl-L-4-thiazolidinecarboxylic acid hydrochloride

A solution of 1.21 g (3.63 mmoles) of tert.butoxycarbonyl-L-alanyl-2-hydroxymethyl-L-4-thiazolidinecarboxylic acid in 20 ml of ethyl acetate saturated with gaseous HCl at 0°C is stirred at ambient temperature for 15 minutes, then evaporated under vacuum. The residue is taken up in ethyl acetate, and the solvent evaporated under vacuum, repeating the operation several times in order to remove the last traces of HCl. 0.87 g of the required product are obtained (yield 89%).

15d) N-(1-carboxy-3-phenylpropyl)-L-alanyl-2-hydroxymethyl-L-thiazolidinecarboxylic acid

0.87 g (3.22 mmoles) of L-alanyl-2-hydroxymethyl-L-4-thiazolidinecarboxylic acid hydrochloride and 2.86 g (16.1 mmoles) of benzylpyruvic acid are dissolved in 30 ml of water and the pH adjusted to 7.5 with 2N NaOH. 1.09 g (17.7 mmoles) of sodium cyanoborohydride are added to the solution and the mixture stirred at ambient temperature for 24 hours. 50 ml of DOWEX 50W resin in $H^+$ form are added, and the mixture after stirring under vacuum to decompose the hydride residue is fed to a chromatograph column containing 40 ml of the same resin. It is eluted with water until neutral, and the product is then eluted with a 2% solution of pyridine in $H_2O$. The product-rich fractions are combined, concentrated under vacuum and further purified by chromatography over DOWEX AG1 resin in $HCOO^-$ form, eluting with a linear gradient of H-COOH(0-1N). (yield 23%) 0.29 g of the required product are recovered by lyophilisation

$^1H$-NMR($D_2O$): $\delta$ 1.65 (3H, d, $CH_3$), 2.40 (2H, m, $\underline{CH_2}$-CH-NH), 2.95 (2H, m, $\underline{CH_2}$-$C_6H_5$), 3.50-4.00 (4H, m, $CH_2O$, $CH_2S$), 4.25-4.70 (2H, m, NCH-COO, OOC-$\underline{CH}$-NH), 5.10-5.75 (2H, m, CHS, $\underline{CH}$-$CH_3$), 7.45 (5H, s, $C_6H_5$).

Mass spectrum: m/e absent ($M^+$), 378, 360, 347, 333, 206, 160, 91, 86.

EXAMPLE 16

N-(1-carboxy-3-phenylpropyl)-L-alanyl-2-hydroxyethyl-L-4-thiazolidinecarboxylic acid

16a) 2-hydroxyethyl-L-4-thiazolidinecarboxylic acid

5.26 g (30 mmoles) of L-cysteine-hydrochloride monohydrate are added to 15 ml of an aqueous solution of 2.22 g (30 mmoles) of 3-hydroxypropionaldehyde, and the mixture is stirred for 2 hours at ambient temperature. After adjusting the pH to 6.0 with 2N NaOH and adding 15 ml of methanol, the precipitate is collected by filtration and dried. 4.30 g of the required product are obtained (yield 81%).

Mass spectrum: m/e absent ($M^+$), 133, 132, 88, 87, 86, 61, 44, 30.

16b) 2-hydroxyethyl-L-4-thiazolidinecarboxylic acid methyl ester

The 2-hydroxymethyl-L-4-thiazolidinecarboxylic acid is replaced in the procedure of example 14b by 2-hydroxyethyl-L-4-thiazolidinecarboxylic acid, to give the required product.

16c) Tert.butoxycarbonyl-L-alanyl-2-hydroxyethyl-L-4-thiazolidinecarboxylic acid methyl ester

The 2-hydroxymethyl-L-4-thiazolidinecarboxylic acid methyl ester is replaced in the procedure of example 15a by 2-hydroxyethyl-L-4-thiazolidinecarboxylic acid methyl ester, to give the required product.

16d) Tert.butoxycarbonyl-L-alanyl-2-hydroxyethyl-L-4-thiazolidinecarboxylic acid

The required product is obtained by basic hydrolysis of tert. butoxycarbonyl-L-alanyl-2-hydroxyethyl-L-4-thiazolidinecarboxylic acid methyl ester, with the procedure of Example 15b.

16e) L-alanyl-2-hydroxyethyl-L-4-thiazolidinecarboxylic acid hydrochloride

Tert.butoxycarbonyl-L-alanyl-2-hydroxyethyl-L-4-thiazolidinecarboxylic acid is used as the starting substance in the procedure of example 15c, to give the required product.

16f) N-(1-carboxy-3-phenylpropyl)-L-alanyl-2-hydroxyethyl-L-4-thiazolidinecarboxylic acid

The L-alanyl-2-hydroxymethyl-L-4-thiazolidinecarboxylic acid hydrochloride is replaced in the procedure of example 15d by L-alanyl-2-hydroxyethyl-L-4-thiazolidinecarboxylic acid hydrochloride, to give the required product.

EXAMPLE 17

N-(1-carboxy-3-phenylpropyl)-L-alanyl-2-tert.butoxy-L-4-thiazolidinecarboxylic acid

17a) 2-tert.butoxy-L-4-thiazolidinecarboxylic acid

A mixture of 3.06 g (30 mmoles) of formic acid tert.butyl ester and 5.26 g (30 mmoles) of L-cysteine-hydrochloride monohydrate is stirred for 24 hours at a bath temperature of 100°C. A solid separates on cooling and adding 15 ml of ethanol, and is collected by filtration and dried to give

4.61 g of the required product (yield 75%).

$^1$H-NMR ($D_2O$): δ 1.80 (9H, s, $(CH_3)_3C$), 3.23 (2H, m, $CH_2$), 4.00 (1H, m, CHCOO).

17b) <u>2-tert.butoxy-L-4-thiazolidinecarboxylic acid methyl ester</u>

A solution of $CH_2N_2$ in ethyl ether is added drop by drop under an inert atmosphere to a stirred solution, cooled to 0°C, of 2.2 g (1.07 mmoles) of 2-tert.butoxy-L-4-thiazolidinecarboxylic acid in 15 ml of methanol, until a persistent yellow colouration is obtained. The mixture is evaporated to dryness under vacuum to give a residue which is suspended in 20 ml of a saturated solution of $NaHCO_3$, and extracted with ethyl ether (3 x 50). The ether phase is dried ($MgSO_4$) and evaporated to dryness under vacuum to give 1.6 g of the required product (yield 72%).

17c) <u>Tert.butoxycarbonyl-L-alanyl-2-tert.butoxy-L-4-thiazolidine-carboxylic acid methyl ester</u>

The 2-hydroxymethyl-L-4-thiazolidinecarboxylic acid methyl ester is replaced in the procedure of example 15a by 2-tert.butoxy-L-4-thiazolidinecarboxylic acid methyl ester, to give the required product.

17d) <u>Tert.butoxycarbonyl-L-alanyl-2-tert.butoxy-L-4-thiazolidine-carboxylic acid</u>

The required product is obtained by basic hydrolysis of tert.butoxycarbonyl-L-alanyl-2-tert.butoxy-L-4-thiazolidinecarboxylic acid methyl ester by the procedure of example 15b.

17e) <u>L-alanyl-2-tert.butoxy-L-4-thiazolidinecarboxylic acid hydrochloride</u>

Tert.butoxycarbonyl-L-alanyl-2-tert.butoxy-L-4-thiazolidinecarboxylic acid is used as the starting substance in the procedure of example 15c to give the required product.

17f) N-(1-carboxy-3-phenylpropyl)-L-alanyl-2-tert.butoxy-L-4-thiazolidinecarboxylic acid

The L-alanyl-2-hydroxymethyl-L-4-thiazolidinecarboxylic acid is replaced in the procedure of example 15d by L-alanyl-2-tert.butoxy-L-4-thiazolidinecarboxylic acid hydrochloride to give the required product.

EXAMPLE 18

N-(1-carboxy-3-phenylpropyl)-L-alanyl-2-carboxy-L-4-thiazolidinecarboxylic acid

18a) 2-carboxy-L-4-thiazolidinecarboxylic acid

7.72 g (44 mmoles) of L-cysteine hydrochloride monohydrate are added to 25 ml of an aqueous solution of 4.99 g (44 mmoles) of glyoxylic acid sodium salt, monohydrate, and the mixture is stirred for 3 hours at ambient temperature. A solid separates on cooling with an ice bath, and is collected by filtration and dried to give 7.0 g of the required product (yield 90%).

$^1$H-NMR(d-DMSO): δ 2.55-3.70 (2H, m, $CH_2$), 3.83 (1H, m, CH-$CH_2$), 5.49 (1H, s, CHS).

Mass spectrum: m/e 177 ($M^+$) 159, 132, 86, 59, 45.

18b) 2-methoxycarbonyl-L-4-thiazolidinecarboxylic acid methyl ester

The 2-hydroxymethyl-L-4-thiazolidinecarboxylic acid is replaced in the procedure of example 14b by 2-carboxy-L-4-thiazolidinecarboxylic acid to give the required product.

18c) Tert.butoxycarbonyl-L-alanyl-2-methoxycarbonyl-L-4-thiazolidinecarboxylic acid methyl ester

The 2-hydroxymethyl-L-4-thiazolidinecarboxylic acid methyl ester is replaced in the procedure of example 15a by 2-methoxycarbonyl-L-4-thiazolidinecarboxylic acid methyl ester to give the required product.

18d) Tert.butoxycarbonyl-L-alanyl-2-carboxy-L-4-thiazolidinecarboxylic acid

The required product is obtained by basic hydrolysis of tert.butoxycarbonyl-L-alanyl-2-methoxycarbonyl-L-4-thiazolidinecarboxylic acid methyl ester by the procedure of example 15b.

18e) L-alanyl-2-carboxy-L-4-thiazolidinecarboxylic acid hydrochloride

Tert.butoxycarbonyl-L-alanyl-2-carboxy-L-4-thiazolidinecarboxylic acid is used as the starting substance in the procedure of example 15c to give the required product.

18f) N-(1-carboxy-3-phenylpropyl)-L-alanyl-2-carboxy-L-4-thiazolidinecarboxylic acid

The L-alanyl-2-hydroxymethyl-L-4-thiazolidinecarboxylic acid hydrochloride is replaced in the procedure of example 15d by L-alanyl-2-carboxy-L-4-thiazolidinecarboxylic acid hydrochloride, to give the required product.

0115091

CLAIM

N-carboxyacyl derivatives of cyclic amino acids useful as anti-hypertensives, of general formula

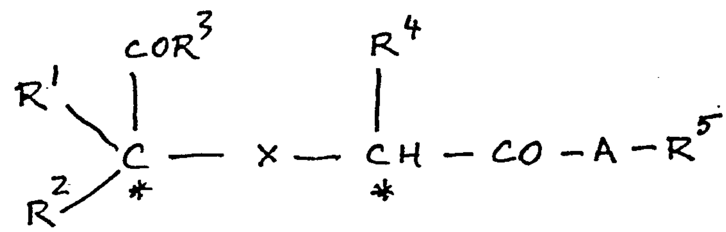

(I)

in which $R^1$ and $R^4$, which can be equal or different, are hydrogen, alkyl, haloalkylene, hydroxyalkylene, alkyloxyalkylene, aryloxyalkylene, aminoalkylene, mono or di-alkylaminoalkylene, acylaminoalkylene, arylaminoalkylene, guanidinoalkylene, mercaptoalkylene, alkylthioalkylene, arylthioalkylene, carboxyalkylene, alkyloxy-carbonylalkylene, carbamoylakylene, aryl or alkyl substituted with an aryl or heterocyclic radical, and in which the alkyl portion can also comprise an amino or acylamino substituent;

$R^2$ is hydrogen, alkyl, aryl or arylalkylene;

$R^3$ and $R^5$, which can be equal or different, are hydroxy, alkyloxy, aryloxy, arylalkyleneoxy, amino, mono or di-alkylamino, arylalkyl-eneamino, hydroxyamino, dialkylaminoalkyleneoxy, acylaminoalkylene-oxy or acyloxyalkyleneoxy;

X is $-\overset{\overset{R^6}{|}}{C}H-$ or $-\overset{\overset{R^7}{|}}{N}-$ where $R^6$ and $R^7$, which can be equal or different, are hydrogen, alkyl or arylalkylene;

A is the residue

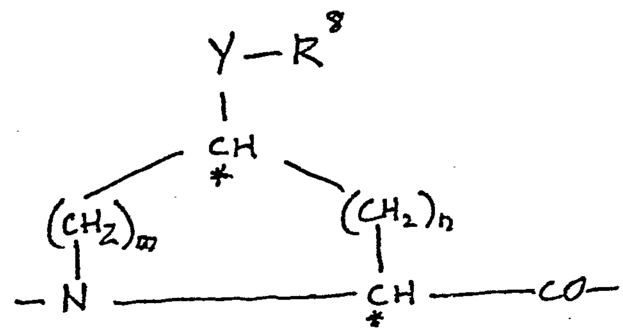

where n and m are 1 or 2 and m+n is 2 or 3, Y is either -O- or -S- and $R^8$ is aryl, a heterocyclic radical, acyl, arylsulphonyl, alkylsulphonyl, trialkylsilyl, nitro, cyclic saturated alkyl, cyclic or non-cyclic unsaturated alkyl, haloalkylene, hydroxyalkylene, alkyloxyalkylene, mercaptoalkylene, alkylthioalkylene, aminoalkylene, mono or di-alkylaminoalkylene, guanidinoalkylene, arylalkylene or alkyl substituted with a heterocyclic radical; when Y is -S-, $R^8$ can also be hydrogen or non-cyclic saturated alkyl;

A can also be the residue

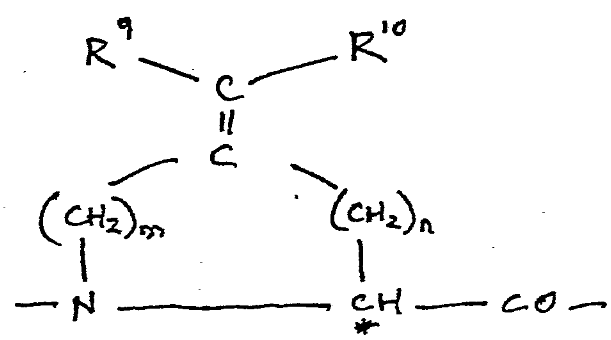

where m and n are 1 or 2 and m+n is 2 or 3;

$R^9$ and $R^{10}$, which can be equal or different, are hydrogen, alkyl, haloalkylene, hydroxyalkylene, alkyloxyalkylene, aryloxyalkylene, aminoalkylene, mono or di-alkylaminoalkylene, acylaminoalkylene, arylaminoalkylene, guanidinoalkylene, mercaptoalkylene, alkylthioalkylene, arylthioalkylene, carboxyalkylene, alkyloxycarbonylalkylene, carbamoylalkylene, aryl or alkyl substituted with an

aryl or heterocyclic radical and in which the alkyl portion can also comprise an amino or acylamino substituent;

A can also be the residue

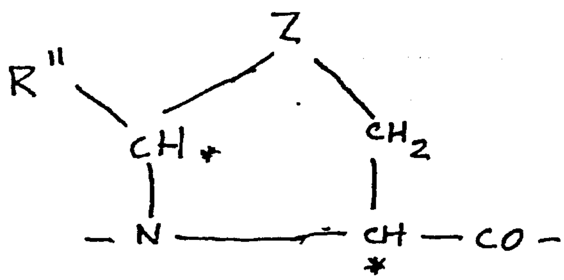

where Z is -S-, 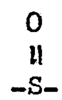 or 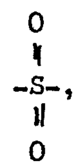,

and $R^{11}$ is hydroxy, alkyloxy, arylalkyleneoxy, aryloxy, acyloxy, carboxy, alkyloxycarbonyl, arylalkyleneoxycarbonyl, aryloxycarbonyl, aminocarbonyl, mono or di-alkylaminocarbonyl, arylalkyleneamino-carbonyl, arylaminocarbonyl, amino, mono or di-alkylamino, aryl-alkyleneamino, arylamino, hydroxyalkylene, alkyloxyalkylene, arylalkyleneoxyalkylene, aryloxyalkylene, aminoalkylene, mono or di-alkylaminoalkylene, or arylaminoalkylene;

the asterisks indicating asymmetric carbon atoms;

and the pharmaceutically acceptable salts of said derivatives.

European Patent Office

# EUROPEAN SEARCH REPORT

0115091

Application number

EP 83 20 0158

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | EP-A-0 049 589 (SQUIBB) *Page 41, examples 42-49; page 43, examples 61-64; page 32, example 21A* | 1 | C 07 C 103/52<br>A 61 K 37/02<br>C 07 D 207/16<br>C 07 D 277/06 |
| | --- | | |
| X | US-A-4 154 937 (SQUIBB) *Column 10, example 22* | 1 | |
| | --- | | |
| X | EP-A-0 052 991 (SQUIBB) *Page 42, example 9; page 47, example 25; page 48, examples 28-30; page 58, example 68* | 1 | |
| | --- | | |
| D,X | EP-A-0 012 401 (MERCK) *Page 72, example 106* | 1 | |
| | ----- | | TECHNICAL FIELDS SEARCHED (Int. Cl. 3) |
| | | | C 07 C 103/00<br>A 61 K 37/00<br>C 07 D 207/00<br>C 07 D 277/00 |

The present search report has been drawn up for all claims

| Place of search | Date of completion of the search | Examiner |
|---|---|---|
| THE HAGUE | 10-03-1983 | GRAMAGLIA R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82